# EUROPEAN PATENT SPECIFICATION

(11) **EP 3 840 909 B1**
(45) Date of publication and mention of the grant of the patent: **23.07.2025**
(21) Application number: 19753399.5
(22) Date of filing: 20.08.2019
(51) Int. Cl.: B23K 26/08, B23K 26/364, B23K 26/0622, A61F 2/91, B23K 101/06, B23K 103/08

(54) **METHOD FOR PRODUCING A STENT**
HERSTELLUNGSMETHODE FÜR EINEN STENT
PROCÉDÉ DE FABRICATION D'UN STENT

(30) Priority: 24.08.2018 EP 18190805
(43) Date of publication of application: 30.06.2021
(73) Proprietor: Biotronik AG, 8180 Bülach (CH)
(72) Inventor: REMUND, Stefan, 3005 Bern (CH); KRAMER, Thorsten, 4500 Solothurn (CH); NEUENSCHWANDER, Beat, 3400 Burgdorf (CH); JAEGGI, Beat, 3427 Utzenstorf (CH)
(74) Representative: Biotronik Corporate Services SE
(86) International application number: PCT/EP2019/072236
(87) International publication number: WO 2020/038925

(56) References cited:
- JP-A- 2012 091 186
- US-A1- 2002 108 937
- US-A1- 2007 075 060
- US-A1- 2015 166 394
- US-B2- 6 563 080
- US-B2- 6 563 080
- BRUNO ZBERG ET AL: "MgZnCa glasses without clinically observable hydrogen evolution for biodegradable implants", NATURE MATERIALS, vol. 8, no. 11, 27 September 2009 (2009-09-27), GB, pages 887 - 891, XP055121391, ISSN: 1476-1122, DOI: 10.1038/nmat2542

## Description

The present invention relates to a method for producing a stent. The present invention is described primarily on the basis of the example of a method for producing a stent from a magnesium alloy. However, the present invention is not limited to this application, but is suitable for producing an implant from any biodegradable material. A biodegradable material within the scope of this application shall be understood to mean a material which is broken down (degraded) in the body. Implants formed from biodegradable material (biodegradable implants) maintain their structural integrity over a period of time lasting from hours/days, through months, to several years, and are then broken down (resorbed) by the body.

Biodegradable materials of this kind are known to a person skilled in the art, for example some metal alloys, such as magnesium, iron or zinc alloys, or degradable polymers, such as polylactides.

In manufacturing processes of this kind, laser radiation is often used to cut the stent, wherein in particular pulsed laser radiation is used for material removal/cutting.

The pulses are described by the pulse duration and their pulse energy. The pulse duration is typically specified as the Full Width at Half Maximum (FWHM), wherein the width of the pulse is measured at half pulse height. For ultra-short pulses with pulse durations in the range of from a few femtoseconds to a few picoseconds, the pulse shape can be considered to be time-symmetrical

The energy of a pulse is calculated formally by the integration of the temporal course of the pulse power (the pulse shape) over time and is specified in joules (J), millijoules (mJ) or microjoules (µJ). It can also be calculated as an averaged value from the quotient of the mean power and the repetition rate.

The repetition rate or also pulse sequence frequency defines how many individual pulses per unit of time are emitted by the laser. It is specified in hertz (Hz), kilohertz (kHz) or megahertz (MHz).

Instead of an individual pulse, it is possible in the case of some laser beam sources, at a given repetition rate, to divide the pulse energy of an individual pulse into a sequence of individual pulses, or what are known as pulse bundles or pulse bursts.

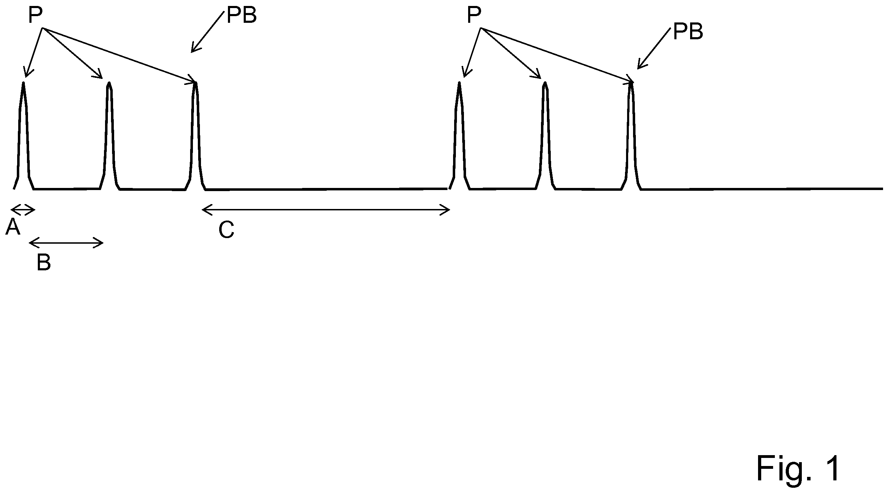

A pulse bundle or pulse burst (PB) is a sequence of individual pulses which consists of at least two successive individual pulses (P), wherein the time interval (B) of the individual pulses of a pulse bundle is shorter than the time interval (C) of two pulse bundles, i.e. the reciprocal value of the repetition rate of the laser. (Fig. 1). In the case of a pulse bundle the time interval between each two adjacent pulses of a pulse bundle is shorter than the time interval between the last pulse of a pulse bundle and the first pulse of the next pulse bundle. Within the scope of this application the terms pulse bundle and pulse burst are used synonymously.

Depending on the structure of the laser, in the case of ultra-short-pulsed laser beam sources a distinction is made between Master-Oscillator-Power-Amplifier (MOPA) systems and Regenerative Amplifier (Regen) systems. The pulse bundle for MOPA systems is generated in what is known as the pulse picker, which not only allows an individual pulse to pass, but also a plurality of pulses (typically 2 to 10) with the selected repetition rate. The time interval between the pulses in the pulse bundle therefore corresponds to the inverse of the frequency of the laser oscillator (Master Oscillator) and lies typically in the range of from 20 MHz to 100 MHz. Such laser sources nowadays already reach frequencies in the GHz range. The oscillator frequency is therefore much higher than typical repetition rates of less than 1 MHz, which are currently used for processing. Expressed more simply, the laser source produces pulsed laser radiation with a high frequency. The pulse picker selects, from a continuous sequence of pulses, the individual pulse bundles with the desired repetition rate of the laser and the individual pulses of a pulse bundle. The pulse picker thus defines the repetition rate of the laser (time interval of the pulse bundles) and the number of pulses of a pulse bundle.

The individual pulses or pulse bundles are then amplified in the amplifier (Power Amplifier). In modern ultra-short-pulse laser systems there is the possibility to adjust the amplification factor individually for each pulse of the pulse bundle. There is thus in particular also the possibility to set the amplification of a pulse to zero and to thus blend out the pulse. The individual pulse number and the time interval between the individual pulses of a pulse bundle are thus controlled. In principle, each pulse bundle can thus be formed individually.

Ultra-short-pulse laser systems are generally used for material removal (ablation). The ablation rate in this case denotes the ablated volume per unit of time and is specified typically in cubic millimetres per minute (mm³/min).

The ablation efficiency or the specific ablation rate is understood to mean the ablation rate based on the introduced mean laser power, that is to say the ablated volume per energy unit. It is therefore specified in mm³/(min W) or also in µm³/(µJ).

During the processing with laser radiation with individual pulses at a selected repetition rate, the maximum ablation efficiency is delimited, amongst other things, by the creation of zones and craters on the workpiece surface, which negatively influence the absorption of the laser radiation. The ablation process, in the worst-case scenario, can come to a complete halt.

The use of individual pulses at a moderate medium power and at not-too-high repetition rates generally leads to the best-possible surface quality in almost all examined materials.

Generally, it is desirable in the case of laser beam ablation processes of this kind to increase the ablation rate whilst maintaining a constant surface quality. In addition, it is expedient and desirable at the same time not to reduce, or even increase, the ablation efficiency.

An increased ablation rate, alongside a constant quality, can be achieved, however, only if the repetition rate of the laser and thus its mean power are increased accordingly. Such an increase is delimited in particular by two factors. On the one hand the mean power in the case of commercial ultra-short-pulsed laser beam sources, which are usually used, is limited to approximately 100 W, and on the other hand a number of tests have shown that, when the repetition rate is increased, the subsequent pulses are influenced increasingly for example by what is known as shielding, i.e. the interaction or partial shielding of the laser radiation of a pulse with the already ablated material of the previous pulse, and therefore the ablation efficiency decreases.

US 2002/0108937 A1 discloses a desired pattern which may be cut into a stent preform by impinging a laser beam onto the stent preform. The laser beam is formed using a laser system comprising a resonator cavity for resonating laser radiation, a gain medium contained in the resonator cavity, a pump for periodically pumping the gain medium and an electro-optical modulator in communication with the resonator cavity. The laser system produces a radiation pulse for each pump period. Each radiation pulse is modulated with the electro-optical modulator to produce a pulse train of ordered pulses of radiation. Each pulse train is output from the optical cavity as an output laser beam which is directed at the stent preform to cut a desired pattern in the stent preform.

US 2007/0075060 Al discloses a method of manufacturing a medical device from a workpiece. The method begins by generating a pulsed beam of radiation from a radiation source. The pulsed radiation beam is characterized by a prescribed pulse frequency. The pulsed radiation beam is directed onto the workpiece and the workpiece is moved relative to the radiation source so that a prescribed pattern is cut in the workpiece by the pulsed radiation beam. The prescribed pulse frequency is adjusted based on a change in a parameter pertaining to the relative motion of the workpiece.

JP 2012-91186 A relates to a laser processing apparatus that performs laser processing on the surface of a cylindrical pipe-shaped object, and an irradiation position control method that matches the irradiation position of laser light in the laser processing apparatus with an appropriate position of the object to be processed.

The object of the invention is therefore to create a method for producing a stent from a biodegradable material which has an increased ablation efficiency This problem is solved by a method having the features of claim 1. Advantageous embodiments of the invention are specified in the dependent claims and will be described hereinafter.

According to claim 1 a method for producing a stent, is disclosed, having the steps of
- providing a stent preform formed from a biodegradable material,
- introducing recesses into stent preform by laser beam ablation,
**characterised in that**
the stent preform is acted on by laser radiation in the form of pulse bundles (PB) in order to introduce the recesses, wherein each pulse bundle (PB) comprises at least two pulses (P), wherein the time interval (B) between each two adjacent pulses (P) of a pulse bundle (PB) lies in a range of 10 ns to 50 ns, wherein the pulse duration (A) of each pulse (P) of a pulse bundle (PB) lies in the range of from 0.200 ps to 20 ps, and wherein the pulse sequence frequency lies in the range of from 0.1 MHz to 20 MHz.

Within the frame of this application laser beam ablation means the use of a laser beam to form a recess in a workpiece. Thereby the thickness of the workpiece is reduced by the recess. This is in contrast to laser beam cutting where the laser beam is used to cut through a workpiece.

Typically a stent comprises a general cylindrical body comprising various meandering struts. If laser beam cutting is used to produce such a stent from a cylindrical workpiece, several cut-outs through-openings are made in the cylindrical workpiece. The remaining material forms the struts of the stent. The laser beam cuts through the cylindrical workpiece. If laser beam ablation is used to produce such a stent, a series of recesses are created in the cylindrical workpiece. The laser beam ablation provides a first recess at a first position. Thereby the thickness of the workpiece is reduced at the first position. At a later point in time a second recess is provided at the same first position, which further reduces the thickness at the first position. This procedure is repeated layer by layer until there is no further material at the first position. Thereby a cut-out through-opening is produced. The remaining material of the cylindrical workpiece forms the struts.

In accordance with an embodiment of the invention, the degradable material is magnesium alloy.

It has surprisingly been found, with regard to the ablation of magnesium alloys, that in particular it is possible to respond to the interaction of successive individual pulses signifying a reduction of the ablation efficiency or a reduction of the processing quality by the use of pulse bundles (pulse bursts). The individual pulses within a pulse bundle can be set individually in respect of their magnitude, i.e. in respect of the energy of each individual pulse, and time interval (in multiples of the inverse of the frequency of the laser oscillator) such that not only is the negative influence compensated, but the ablation efficiency can be increased. Since, in addition, the repetition rate does not have to be increased to the same extent as in the case of individual pulses, the interactions between the individual pulse bundles can be reduced or even completely avoided.

Due to the adapted use of pulse bundles during the ablation with ultra-short-pulsed laser radiation, in particular of metals, the specific ablation rate, also referred to as ablation efficiency, i.e. the volume ablated per unit of time and introduced mean laser power, can be increased.

In the case of ablation with ultra-short-pulsed laser radiation, various effects occur at the workpiece surface and over said surface and influence the ablation process. These can be in particular modifications of the surface or shieldings by ablated particles. By selective use of pulse bursts in a configuration that counteracts the mechanisms which negatively influence the ablation process, the ablation efficiency in the case of magnesium alloys can be increased, what's more even beyond the value of an individual pulse.

A comparable effect is produced with other biodegradable materials, in particular with biodegradable polymers, such as polylactides, or other metal alloys, such as iron or zinc alloys.

In accordance with a preferred embodiment it is provided that each pulse bundle of the laser radiation used for the ablation has three pulses, in particular precisely or merely three pulses. In this regard it has been demonstrated that, in the case of a magnesium alloy, compared to an individual pulse the use of a pulse bundle with three pulses increases the ablation efficiency from 0.19 mm³/(min•W) by 68% to 0.32 mm³/(min•W).

In accordance with a further preferred embodiment it is provided that each pulse bundle of the laser radiation used for ablation has four pulses, in particular precisely or merely four pulses. In this regard it has been demonstrated that, in the case of a magnesium alloy, compared to an individual pulse the use of a pulse bundle with four pulses increases the ablation efficiency from 0.19 ₘₘ³/(min•W) by 142% to 0.46 mm³/(min•W).

In accordance with a further preferred embodiment it is provided that each pulse bundle of the laser radiation used for ablation has five pulses, in particular precisely or merely five pulses. In this regard it has been demonstrated that, in the case of a magnesium alloy, compared to an individual pulse the use of a pulse bundle with five pulses increases the ablation efficiency from 0.19 mm³/(min•W) by 195% to 0.56 mm³/(min•W).

According to the invention, the time interval between each two adjacent pulses of a pulse bundle lies in a range of 10 ns to 50 ns (100 MHz to 20 MHz), and in particular is 12 ns.

The use of pulse bundles in which adjacent pulses of the pulse bundle have a time interval of approximately 12 ns leads advantageously to a local melting, of limited depth, of the surface of the material of the stent preform, and therefore already the first unevennesses and inhomogeneities are compensated and the formation of cones and craters is prevented.

According to the invention, the pulse duration of the pulses of a pulse bundle lies in the range of from 0.200 ps to 20 ps. According to a preferred embodiment, the pulse duration of the pulses of a pulse bundle is in particular 10 ps. Pulse durations for the pulses of a pulse bundle likewise advantageously lie in the range of from 0.2-0.5 ps, 0.8-0.9 ps, 6-12 ps or around 20 ps.

According to the invention, the repetition rate (pulse sequence frequency) of the pulse bundles lies in a range of from 0.1 MHz to 20 MHz. According to a preferred embodiment, the repetition rate of the pulse bundles is 500 kHz. Repetition rates for the pulse bundles are likewise advantageously in the region of 0.1-1 MHz, 2 MHz or 8 MHz.

It is furthermore provided in accordance with an embodiment of the invention that the wavelength of the laser radiation lies in the infrared range of from 1000 nm to 1500 nm, in particular is 1026-1090 nm. In some embodiments, however, it may also be advantageous to use a laser source with a laser radiation in the range of 513-545 nm or 342-363 nm.

Furthermore, the stent preform, in accordance with an embodiment can be tubular and extends along a longitudinal axis or cylinder axis.

In accordance with an embodiment of the invention, the stent preform, as it is acted on with the laser radiation in order to form the recesses, is rotated and/or is moved in the direction of the longitudinal axis.

In accordance with an embodiment of the invention it is also possible (in particular as an alternative to a movement of the stent preform, along its longitudinal axis) that the laser radiation in order to form the recesses is moved along the longitudinal axis (for example a head of a cutting tool emitting the laser light can be moved along the longitudinal axis of the stent preform).

The present invention is particularly suitable for cutting and structuring vascular supports/stents. Since the processing is performed layer-by-layer by material ablation, in principle any geometry advantageously can be processed.

With regard to the processing of vascular supports/stents, the increase in efficiency according to the invention results in a decisive reduction of the processing duration, with a simultaneous increase in the flexibility as compared to the conventional thermal laser beam cutting.

Further features and advantages of the invention will be explained in the description of the figures depicting exemplary embodiments of the invention, provided with reference to said figures, in which:
- Fig. 1: shows a schematic depiction of pulse bundles (pulse bursts) with three pulses; and
- Fig. 2: shows the ablation efficiency via the energy irradiated into the material per area ϕ₀ in the case of a magnesium alloy according to the invention and in the case of pulse bundles having three, four and five pulses as compared to an individual pulse.

Figure 1 shows a schematic depiction of pulse bundles (PB) with three pulses P as can be used to cut a stent preform formed from a magnesium alloy of the type described herein. The pulse bundle operation of the laser used for ablation/cutting is characterised in that the time intervals B of two adjacent pulses P of a pulse bundle PB are shorter than the time interval C between the last pulse P of a pulse bundle PB and the first pulse P of the subsequent pulse bundle PB.

In accordance with the invention a laser beam with pulse bundles PB of this kind is directed to the stent preform and a plurality of recesses are cut out or ablated from the stent preform, which in particular is tubular, so as to obtain in particular a stent having a plurality of cells, which are delimited by struts of the stent.

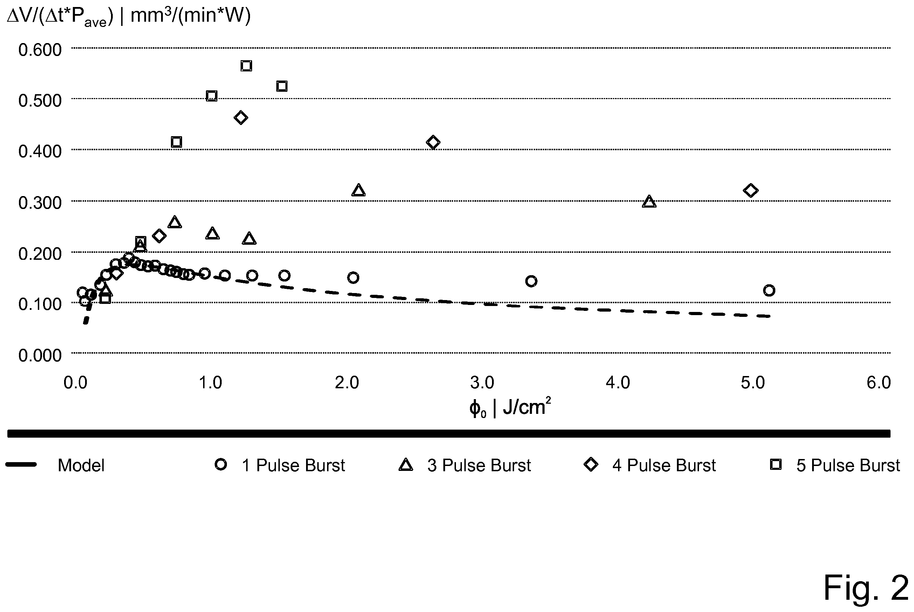

As is shown in Figure 2, the ablation efficiency, which is plotted on the Y-axis or ordinate (the energy irradiated into the stent material per area ϕ₀ is plotted on the abscissa), can be increased when laser radiation with pulse bundles having three, four or five pulses instead of individual pulses is used to ablate the stent material (here for example a magnesium alloy).

The pulse duration A used here for the pulses P of the pulse bundle PB is A = 10 ps. The interval B of the pulses B = 12 ns, and the interval C of the pulse bundles PB is C = 2 µs.

This corresponds to a repetition rate (pulse sequence frequency) of the pulse bundle PB of 500 kHz. Laser radiation of wavelength λ = 1064 nm is used (see Fig. 1).

In principle, commercial laser systems such as LUMENTUM, Amplitude Systémes or Coherent can be used order to produce the pulse bundles PB according to the invention.

## Claims

1. A method for producing a stent, comprising the steps:
- providing a stent preform formed from a biodegradable material,
- introducing recesses into stent preform by laser beam ablation,
**characterised in that**
the stent preform is acted on by laser radiation in the form of pulse bundles (PB) in order to introduce the recesses, wherein each pulse bundle (PB) comprises at least two pulses (P), wherein the time interval (B) between each two adjacent pulses (P) of a pulse bundle (PB) lies in a range of 10 ns to 50 ns, wherein the pulse duration (A) of each pulse (P) of a pulse bundle (PB) lies in the range of from 0.200 ps to 20 ps, and wherein the pulse sequence frequency lies in the range of from 0.1 MHz to 20 MHz.

2. The method according to claim 1, **characterised in that** each pulse bundle (PB) is formed by three pulses (P).

3. The method according to claim 1, **characterised in that** each pulse bundle (PB) is formed by four pulses (P).

4. The method according to claim 1, **characterised in that** each pulse bundle (PB) is formed by five pulses (P).

5. The method according to any one of the preceding claims, **characterised in that** the pulse duration (A) of each pulse (P) of a pulse bundle (PB) is 10 ps.

6. The method according to any one of the preceding claims, **characterised in that** the pulse sequence frequency is 500 kHz to 5 MHz.

7. The method according to any one of the preceding claims, **characterised in that** the wavelength of the laser radiation lies in the infrared range of from 1000 nm to 1500 nm, in particular 1026-1090 nm.

8. The method according to any one of the preceding claims, **characterised in that** the stent preform is tubular and extends along a longitudinal axis.

9. The method according to claim 8, **characterised in that** the stent preform as it is irradiated with the laser radiation in order to form the recesses, is rotated and/or is moved in the direction of the longitudinal axis.

10. The method according to claim 9, **characterised in that** the laser radiation is moved along the longitudinal axis in order to form the recesses.

11. The method according to any one of the preceding claims, wherein the biodegradable material is magnesium alloy.

## Patentansprüche

1. Verfahren zur Herstellung eines Stents, folgende Schritte umfassend:
- Bereitstellen einer Stent-Vorform aus einem biologisch abbaubaren Material,
- Einarbeiten von Vertiefungen in die Stent-Vorform durch Laserstrahl-Ablation,
**dadurch gekennzeichnet, dass**
die Stent-Vorform durch Laserstrahlung in Form von Pulsbündeln (PB) beaufschlagt wird, um die Vertiefungen einzuarbeiten, wobei jedes Pulsbündel (PB) mindestens zwei Pulse (P) umfasst, wobei das Zeitintervall (B) zwischen jeweils zwei benachbarten Pulsen (P) eines Pulsbündels (PB) in einem Bereich zwischen 10 ns und 50 ns liegt, wobei die Pulsdauer (A) jedes Pulses (P) eines Pulsbündels (PB) im Bereich zwischen 0,200 ps und 20 ps und die Pulssequenzfrequenz im Bereich zwischen 0,1 MHz und 20 Mhz liegt.

2. Verfahren nach Anspruch 1, **dadurch gekennzeichnet, dass** jedes Pulsbündel (PB) durch drei Pulse (P) gebildet wird.

3. Verfahren nach Anspruch 1, **dadurch gekennzeichnet, dass** jedes Pulsbündel (PB) durch vier Pulse (P) gebildet wird.

4. Verfahren nach Anspruch 1, **dadurch gekennzeichnet, dass** jedes Pulsbündel (PB) durch fünf Pulse (P) gebildet wird.

5. Verfahren nach einem der vorstehenden Ansprüche, **dadurch gekennzeichnet, dass** die Pulsdauer (A) jedes Pulses (P) eines Pulsbündels (PB) 10 ps ist.

6. Verfahren nach einem der vorstehenden Ansprüche, **dadurch gekennzeichnet, dass** die Pulssequenzfrequenz zwischen 500 kHz und 5 Mhz liegt.

7. Verfahren nach einem der vorstehenden Ansprüche, **dadurch gekennzeichnet, dass** die Wellenlänge der Laserstrahlung im Infrarot-Bereich zwischen 1000 nm und 1500 nm, insbesondere 1026-1090 nm liegt.

8. Verfahren nach einem der vorstehenden Ansprüche, **dadurch gekennzeichnet, dass** die Stent-Vorform röhrenförmig ist und sich entlang einer Längsachse erstreckt.

9. Verfahren nach Anspruch 8, **dadurch gekennzeichnet, dass** die Stent-Vorform, während sie mit der Laserstrahlung bestrahlt wird, um die Vertiefungen auszubilden, rotiert wird und/oder in Richtung der Längsachse bewegt wird.

10. Verfahren nach Anspruch 9, **dadurch gekennzeichnet, dass** die Laserstrahlung entlang der Längsachse bewegt wird, um die Vertiefungen auszubilden.

11. Verfahren nach einem der vorstehenden Ansprüche, wobei das biologisch abbaubare Material Magnesiumlegierung ist.

## Revendications

1. Procédé de production d'un stent, comprenant les étapes consistant à :
- fournir une préforme de stent formée à partir d'un matériau biodégradable,
- introduire des évidements dans la préforme de stent par ablation au faisceau laser,
**caractérisé en ce que**
la préforme de stent est travaillée par rayonnement laser sous la forme de trains d'impulsions (PB) afin d'introduire les évidements, dans lequel chaque train d'impulsions (PB) comprend au moins deux impulsions (P), dans lequel l'intervalle de temps (B) entre chaque ensemble de deux impulsions adjacentes (P) d'un train d'impulsions (PB) est compris dans une plage de 10 ns à 50 ns, dans lequel la durée d'impulsion (A) de chaque impulsion (P) d'un train d'impulsions (PB) est comprise dans la plage allant de 0,200 ps à 20 ps, et dans lequel la fréquence de séquence d'impulsions est comprise dans la plage de 0,1 MHz à 20 MHz.

2. Procédé selon la revendication 1, **caractérisé en ce que** chaque train d'impulsions (PB) est formé de trois impulsions (P).

3. Procédé selon la revendication 1, **caractérisé en ce que** chaque train d'impulsions (PB) est formé de quatre impulsions (P).

4. Procédé selon la revendication 1, **caractérisé en ce que** chaque train d'impulsions (PB) est formé de cinq impulsions (P).

5. Procédé selon l'une quelconque des revendications précédentes, **caractérisé en ce que** la durée d'impulsion (A) de chaque impulsion (P) d'un train d'impulsions (PB) est de 10 ps.

6. Procédé selon l'une quelconque des revendications précédentes, **caractérisé en ce que** la fréquence de séquence d'impulsions est de 500 kHz à 5 MHz.

7. Procédé selon l'une quelconque des revendications précédentes, **caractérisé en ce que** la longueur d'onde du rayonnement laser est comprise dans la plage infrarouge allant de 1000 nm à 1500 nm, en particulier de 1026 à 1090 nm.

8. Procédé selon l'une quelconque des revendications précédentes, **caractérisé en ce que** la préforme de stent est tubulaire et s'étend le long d'un axe longitudinal.

9. Procédé selon la revendication 8, **caractérisé en ce que** la préforme de stent, alors qu'elle est irradiée par le rayonnement laser afin de former les évidements, est tournée et/ou est déplacée dans la direction de l'axe longitudinal.

10. Procédé selon la revendication 9, **caractérisé en ce que** le rayonnement laser est déplacé le long de l'axe longitudinal afin de former les évidements.

11. Procédé selon l'une quelconque des revendications précédentes, dans lequel le matériau biodégradable est un alliage de magnésium.
